# EUROPEAN PATENT SPECIFICATION

(11) **EP 2 603 096 B1**
(45) Date of publication and mention of the grant of the patent: **26.11.2014**
(21) Application number: 11749368.4
(22) Date of filing: 30.07.2011
(51) Int. Cl.: A23L 1/30, A61K 31/575, A61P 1/16

(54) **METHOD FOR TREATING FATTY LIVER DISEASES, IN PARTICULAR NON-ALCOHOLIC STEATOHEPATITIS.**
VERFAHREN ZUR BEHANDLUNG VON FETTLEBERERKRANKUNGEN, IM BESONDEREN NICHT-ALKOHOLISCHE STEATOHEPATITIS
MÉTHODE DE TRAITEMENT DES STÉATOSES HÉPATIQUES ET, EN PARTICULIER, DE LA STÉATOHÉPATITE NON ALCOOLIQUE

(30) Priority: 09.08.2010 EP 10172304
(43) Date of publication of application: 19.06.2013
(73) Proprietor: Universiteit Maastricht, 6211 LK Maastricht (NL); Academisch Ziekenhuis Maastricht, 6229 HX Maastricht (NL)
(72) Inventor: SVERDLOV, Ronit, NL-6228 SB Maastricht (NL); BIEGHS, Veerle, B-3650 Dilsen (BE); GORP, VAN, Patrick, Johannes, Jacobus, NL-6301 LR Valkenburg a/d Geul (NL)
(74) Representative: Habets, Winand
(86) International application number: PCT/EP2011/063173
(87) International publication number: WO 2012/019930

(56) References cited:
- WO-A1-95/15165
- SHIRI-SVERDLOV RONIT ET AL: "Modulating liver inflammation: a crucial role for cholesterol", CHEMISTRY AND PHYSICS OF LIPIDS, vol. 154, no. Suppl. S, August 2008 (2008-08), page S14, XP002604486, & 49TH INTERNATIONAL CONFERENCE ON THE BIOSCIENCE OF LIPIDS; MAASTRICHT, NETHERLANDS; AUGUST 26 -30, 2008 ISSN: 0009-3084
- LI TIANGANG ET AL: "PXR induces CYP27A1 and regulates cholesterol metabolism in the intestine", JOURNAL OF LIPID RESEARCH, vol. 48, no. 2, February 2007 (2007-02), pages 373-384, XP002604487, ISSN: 0022-2275, DOI: 10.1194/JLR.M600282-JLR200
- PETTA S ET AL: "Low vitamin d serum level is related to severe fibrosis and low responsiveness to interferon-based therapy in genotype 1 chronic hepatitis C", HEPATOLOGY, WILLIAMS AND WILKINS, BALTIMORE, MD, US, vol. 51, no. 4, 1 January 2010 (2010-01-01), pages 1158-1167, XP009139741, ISSN: 0270-9139

## Description

### Field of the invention

The invention is in the field of prevention and medical treatment of liver diseases, in particular fatty liver disease, more in particular non-alcoholic fatty liver disease, such as non-alcoholic steatohepatitis.

### Background of the invention

Non alcoholic fatty liver disease (NAFLD) is a condition ranging from benign lipid accumulation in the liver (steatosis) to steatosis combined with inflammation. The latter is referred to as non-alcoholic steatohepatitis (NASH). NASH is viewed as the hepatic event of the metabolic syndrome. Estimates in USA are that 5.7-17% of all adults have NASH, while 17% to 33% of Americans have NAFLD. NASH and NAFLD are frequently reported in both men and women, although it most often appears in women and is especially prevalent in the obese. As obesity and insulin resistance reach epidemic proportions in industrialized countries, the prevalence of both NAFLD and NASH is increasing and is therefore considered as a major health hazard.

The disease termed non-alcoholic steatohepatitis (NASH) was coined by Jurgen Ludwig in 1980 (1). Since NASH's initial clinical recognition, it has become a leading cause of liver disease in our current society (2). NASH occurs in patients that consume little to no alcohol, as the name implies, and is characterized by the presence of steatosis, fat accumulation, and inflammation. This disease is closely associated with metabolic syndrome, insulin resistance, and hypertension (3, 4). The trend for increased cases of obesity also contributes to other metabolic factors such as high plasma lipid levels and diabetes which further complicate NASH. The high prevalence of fatty liver and high fat diets in Western society will, unfortunately, become a significant future source of NASH (5). By 2025 the prevalence of adult obesity could reach 20 to 50% in developed countries. Not only are adults at risk; a trend for higher body mass index (BMI) in children has also been reported (6).

Unmonitored, the advanced progression of NASH may result in permanent hepatocellular damage of the liver with features such as fibrosis, cirrhosis, and even liver cancer (7). Liver transplantation may be necessary in many cases; however, recurrent disease post-liver transplantation is a significant setback and relatively common for NASH derived cirrhosis (8). After liver transplantation, these patients with recurrent NASH are also likely to develop symptoms associated with the metabolic syndrome such as diabetes, dyslipidemia, and weight gain (8, 9).

Surprisingly, though many obese people have a fatty liver, not all will develop NASH. Steatosis, one of the principal features of NASH, is considered a benign and reversible condition (10). Even more interesting are those patients who are not overweight, have normal plasma lipid levels, and do not have diabetes yet still develop NASH. Regrettably, NASH is generally an asymptomatic disease and many patients remain unaware, though some have reported fatigue, malaise, and abdominal discomfort (11). Due to its asymptomatic nature, indications of NASH usually only appear following routine liver function and blood panel tests.

Unfortunately, thus far, there is no cure for NASH. Adding to the negative repercussions of the disease is the reality that current therapy options are very poor. No specific treatment is available, though several recommendations do exist. Depending on the specific circumstances, patients are advised to lose weight, strive for healthier eating habits, and avoid unnecessary medications (2, 10). These recommendations, valid as they may be, do not suffice for the treatment of NASH. Given that NASH is a multifaceted disease comprised of conditions such as obesity, type 2 diabetes, and arthrosclerosis there is a need for a non-invasive quantification of steatosis and inflammation (10). Therapy options for NASH are a major unmet need and more focused investigations are vital to determine the mechanism with which inflammation is triggered.

Steatosis alone is considered a relatively benign and reversible condition. However, the transition towards NASH represents a key step in the pathogenesis, as it will set the stage for further damage to the liver, such as fibrosis, cirrhosis and liver cancer.

Currently, liver biopsy is used in the clinical practice as the primary method for detection of liver inflammation in NASH.

There is no established therapy for patients suffering from NASH. Therapy is, therefore, focused mainly on risk factors, weight reduction and pharmacological intervention. Promising pharmacological treatments have been demonstrated with antioxidants, insulin sensitizers, hepatoprotectants and lipid-lowering agents.

### Summary of the invention

In normal circumstance, cholesterol is hydrolyzed in the lysosomes of Kupffer cells, the resident macrophages of the liver. Only when hydrolyzed does this cholesterol leave the lysosomes into the cytoplasm where it is converted by the enzyme CYP27 to 27-hydroxycholesterol (27-HC). We have found that liver inflammation stems from lysosomal cholesterol accumulation within Kupffer cells. Incubation of fibroblasts with 27-HC was found to decrease endolysosomal cholesterol accumulation. Further animal studies with CYP27-/- knockout mice exhibited reduced lysosomal accumulation and hepatic inflammation.

We show herein that liver inflammation may be treated in a representative animal model by exposing the animal to 27-HC.

The present invention therefore provides methods and compositions for the prevention or treatment of non-alcoholic fatty liver disease (NAFLD) and conditions associated with NAFLD. More in particular, it provides a method for the prevention or treatment of non-alcoholic steatohepatitis, (NASH).

The disclosure provides that such diseases may be prevented or treated by administering a therapeutically effective amount of a composition comprising a compound capable of increasing the intracellular level of 27-hydroxycholesterol (27-HC).

In other terms, the invention relates to a pharmaceutical composition comprising a compound capable of increasing the intracellular level of 27 hydroxycholesterol for use in the treatment of non-alcoholic fatty liver disease.

This invention is also directed to a combination therapy treatment for treating Metabolic Syndrome. The Metabolic Syndrome is a set of metabolic abnormalities including centrally distributed obesity, hyperlipideniia, elevated triglycerides, elevated blood pressure, cardiovascular diseases, Type II Diabetes and NAFLD. As part of a combination therapy regime for the treatment of Metabolic Syndrome, pharmaceutical formulations of 27-HC are used for treating the NAFLD (and NASH), component of Metabolic Syndrome.

### Legend to the figures

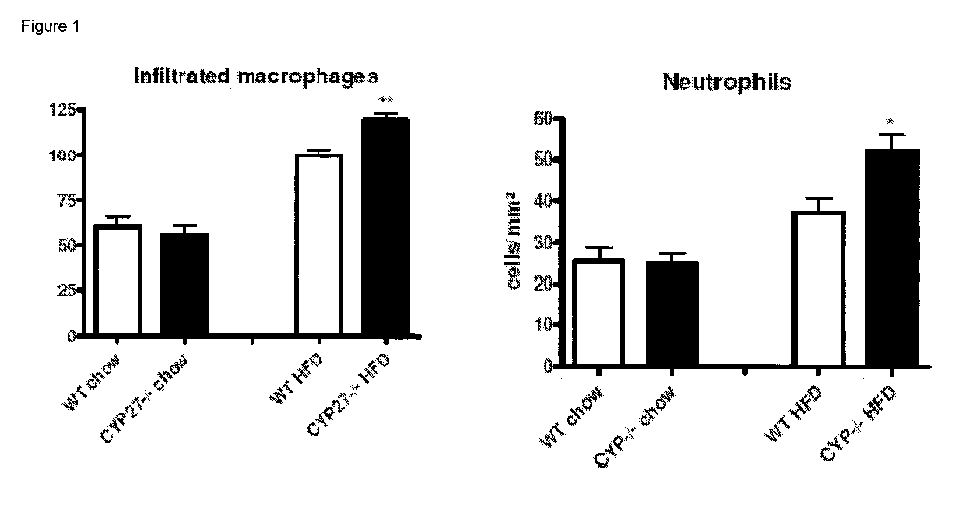

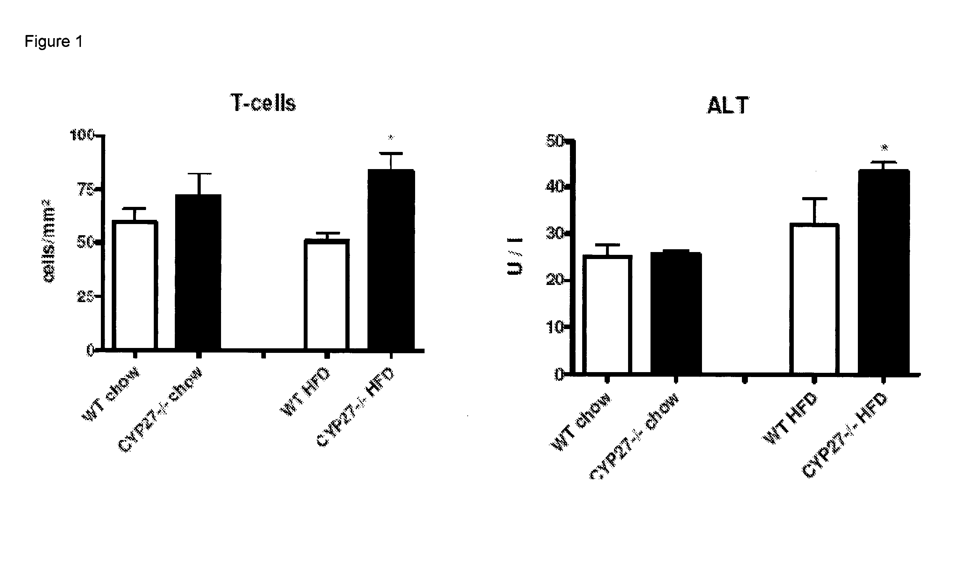

Figure 1: Graphs showing that hepatic inflammation and liver damage is increased in mice transplanted with bone marrow from Cyp27A1 knock out mice as indicated by the increased number of inflammatory cells and ALT levels.

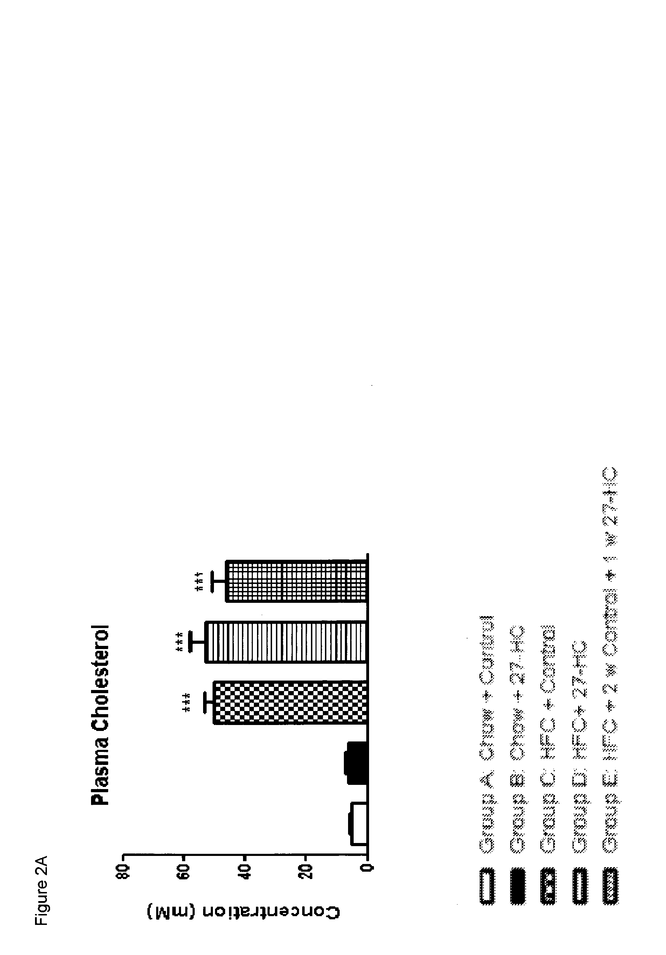

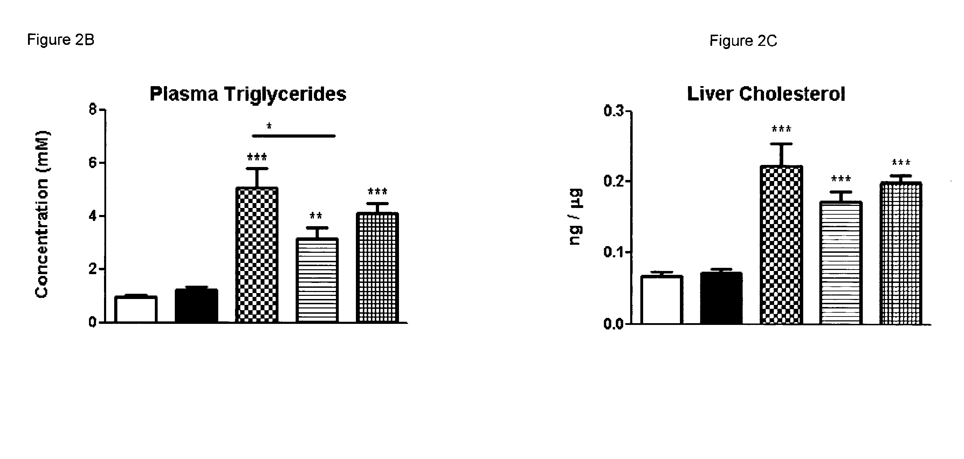

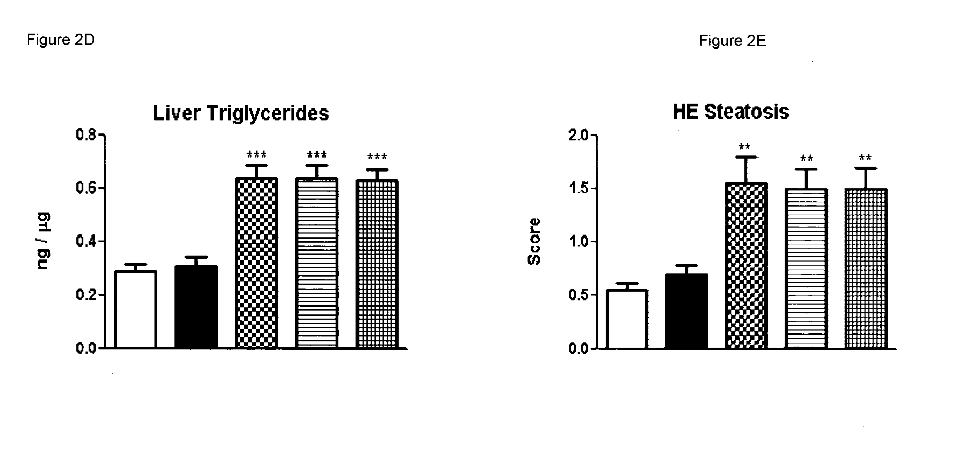

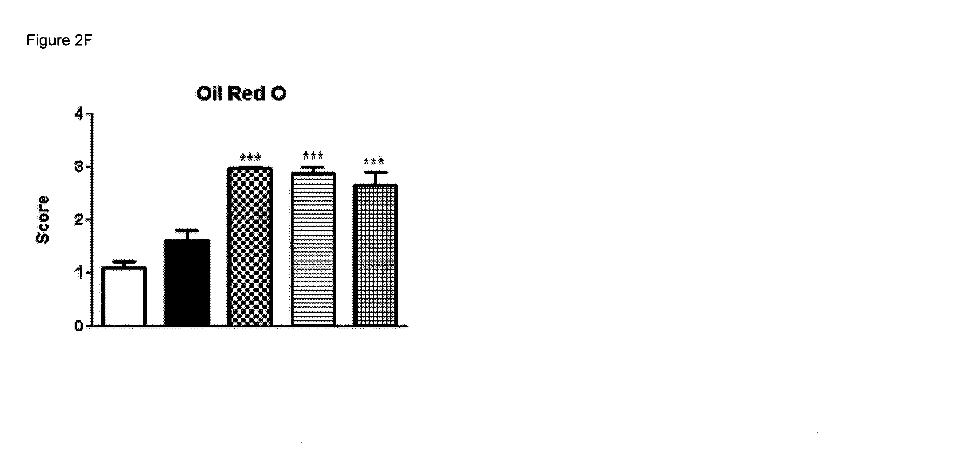

Figure 2: Lipid Measurements. (A, C) Plasma and liver cholesterol measurements. (B, D) Plasma and liver triglycerides levels. The line represents significance between the high fat diet and control injection when compared with the high fat diet and 27-HC injected mice (Group D). (E) Scoring results or steatosis. (F) Scoring outcomes for the oil red O stain. Group A is indicated by the white bars, Group B by black bars, Group C by checkered bars, Group D by striped bars, and Group E by the grey mesh bars. All other asterisks indicate significance between Groups A and B when compared to the three high fat diet groups (Groups C-E), *P< 0.05, **P < 0.01, and ***P <0.001, respectively.

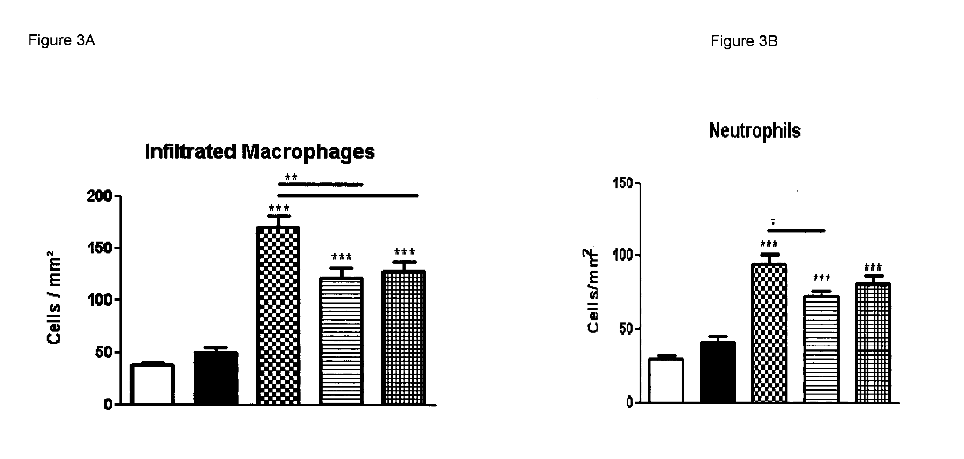

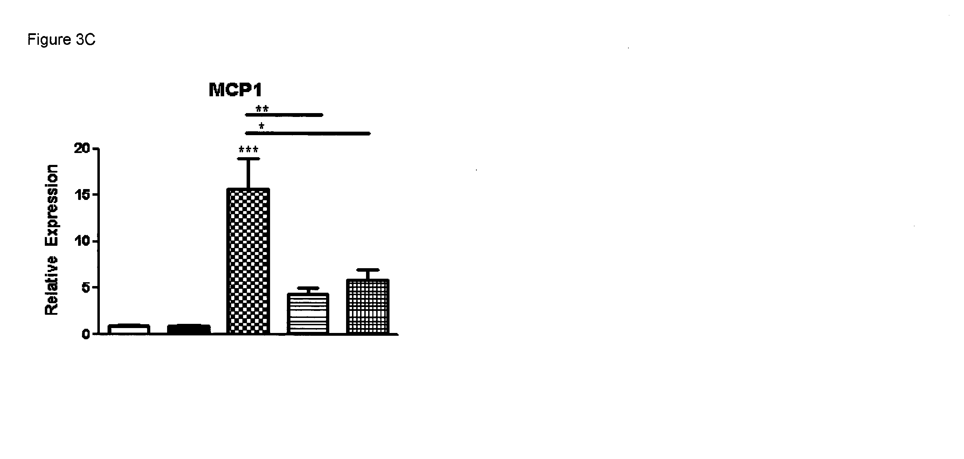

Figure 3: Hepatic Inflammation. (A) Immunohistochemical staining of macrophage infiltration. The lines represent significances between the high fat diet and control injection when compared to the high fat diet and 27-HC injected mice as well as the mice injected with 27-HC only in the last week (Group D, E respectively). (B) NIMP staining for neutrophils. (C) Inflammatory gene expression analyzed with quantitative real time PCR. MCP1 gene expression analysis with significant differences between Group C and Groups D, E. Group A is indicated by the white bars, Group B by black bars, Group C by checkered bars, Group 4 by striped bars, and Group E by the grey mesh bars. All other asterisks indicate significance between Groups A and B when compared to the three high fat diet groups (Groups C-E), *P< 0.05, **P < 0.01, and ***P < 0.001, respectively.

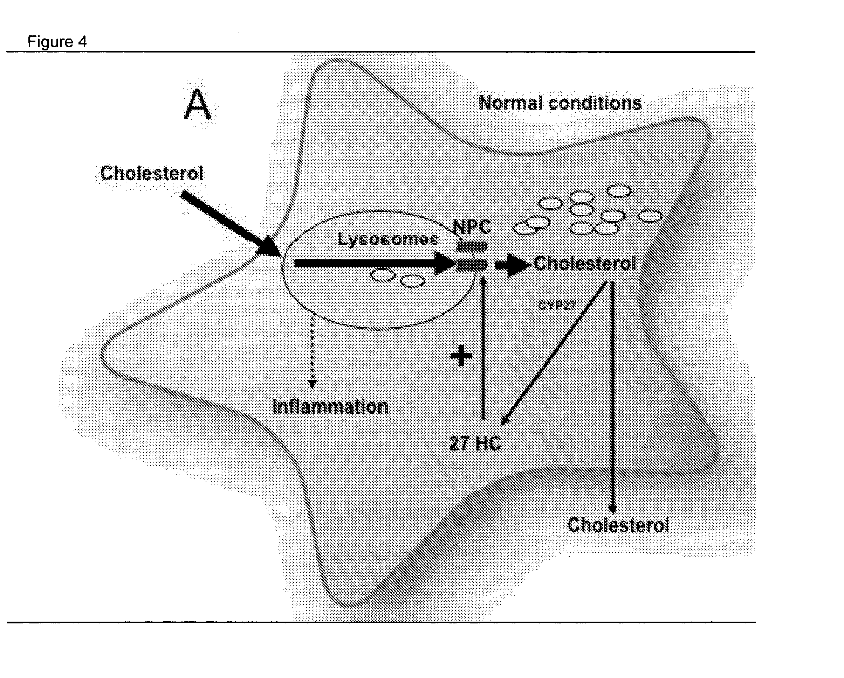

Figure 4: Model showing the role of 27-HC in reducing the levels of lysosomal cholesterol levels.

### Detailed description of the invention

Currently, the mechanisms that drive hepatic inflammation during the progression of NASH are largely unknown. We found that lysosomal fat accumulation in Kupffer cells is the underlying mechanism of inflammation in non-alcoholic fatty liver disease, in particular in non-alcoholic steatohepatitis (NASH).

Our study shows for the first time that liver inflammation is induced when 27-HC levels are decreased. We were also able to show that increased levels of 27-HC remedied the problem of lysosomal cholesterol accumulation and that liver inflammation could be ameliorated or cured by increasing 27-HC levels in vivo.

Hence, the invention relates to a method of reducing liver inflammation in a patient with fatty liver disease by administering an effective amount of a compound capable of increasing the intracellular level of 27-HC.

One way of increasing the intracellular level of 27-HC is to administer a compound comprising 27-HC as an active component. 27-HC may be contained in a pharmaceutically acceptable carrier, and may be administered either orally, parenterally, by injection, intravenous infusion, inhalation, controlled dosage release or by intraperitoneal administration for the treatment or alleviation of NAFLD. The preferred method of administration is orally.

Endogenously produced 27-HC may also be used in a method according to the invention. Generally, such a method involves providing a compound increasing the intracellular level of 27-HC by increasing the in vivo production of 27-HC. Such a compound may be sterol 27-hydroxylase CYP27A1.

Enhancement of naturally occurring 27-HC may also be accomplished by in vivo administration into cells competent for the production of 27-HC, a vector comprising and expressing a DNA sequence encoding biologically active sterol 27-hydroxylase CYP27A1. Inside KCs, the breakdown of free cholesterol by Cyp27A1 results in the formation of 27-hydroxycholesterol. The vector used may be a viral vector, including but not limited to a lentivirus, adenovirus, adeno-associated virus and virus-like vectors, a plasmid, or a lipid vesicle. The vector is taken up by the cells competent for the production of biologically active 27-HC.The DNA sequence is expressed and the biologically active 27-HC is produced.

In one embodiment, the fatty liver disease is non-alcoholic fatty acid liver disease (NAFLD), more in particular non-alcoholic steatohepatitis (NASH

The method according to the invention may be used for the treatment of the diseases mentioned herein but may also be used prophylactic, i.e. in order to prevent the diseases from occurring or ameliorating the inflammatory effects once the disease occurs.

27-HC may be administered via the diet or as a drug or a pro-drug, orally, intravenously or in any other suitable way. Alternatively, the in vivo bioavailability of 27-HC may be increased by interfering with the in vivo pathway that produces 27-HC. For example, the bioavailability of 27-HC may be increased by increasing the activity of CYP 27 or increasing the expression level of CYP 27A1.

If administered as a drug or a food supplement, the 27-HC is to be administered in an effective dose or an effective amount. The phrase "effective dose" or "effective amount" refers to that dose or amount of a substance that produces some desired local or systemic effect at a reasonable benefit/risk ratio applicable to any treatment. The effective amount of such substance will vary depending upon the subject and disease condition being treated, the weight and age of the subject, the severity of the disease condition, the manner of administration and the like, which can readily be determined by one of ordinary skill in the art.

More in particular, the total daily dose of 27-HC is usually in the milligram range per kilogram body weight, such as between 5 and 100 milligram per kilogram body weight, more in particular about 40 milligram per kilogram body weight. In yet another embodiment, the 27-HC composition is administered at a frequency of 4 or less times per day (e.g., one, two or three times per day).

The terms "therapeutic agent," and "active agent" may be used interchangeably and refer to a substance, such as a chemical compound or complex, that has a measurable beneficial physiological effect on the body, such as a therapeutic effect in treatment of a disease or disorder, when administered in an effective amount. Further, when these terms are used, or when a particular active agent is specifically identified by name or category, it is understood that such recitation is intended to include the active agent per se, as well as pharmaceutically acceptable, pharmacologically active derivatives thereof, or compounds significantly related thereto, including without limitation, salts, pharmaceutically acceptable salts, N-oxides, prodrugs, active metabolites, isomers, fragments, analogs, solvates hydrates, radioisotopes, etc.

The term "Metabolic Syndrome" is used to define a set of conditions that places people at high risk for coronary artery disease. These conditions include Type II diabetes, central obesity also known as visceral adiposity, high blood pressure, and a poor lipid profile with elevated LDL ("bad") cholesterol, low HDL ("good") cholesterol, elevated triglycerides. All of these conditions are associated with high blood insulin levels. Associated diseases include NAFLD (especially in concurrent obesity) polycystic ovarian syndrome; hemochromatosis (iron overload); acanthosis nigricans (a skin condition featuring dark patches); Non-alcoholic steatohepatitis (NASH - an extreme form of fatty liver).

The term "NAFLD" (non-alcohol fatty liver disease) refers to a group of conditions where there is an accumulation of excess fat in the liver of people who drink little or no alcohol. In fatty liver disease, fat accumulates in the liver cells. A subgroup of people with NAFLD may have a more serious condition termed non-alcoholic steatohepatitis (NASH). In NASH, fat accumulation is associated with liver cell inflammation and different degrees of scarring. Cirrhosis occurs when the liver sustains substantial damage, and the liver cells are gradually replaced by scar tissue which results in the inability of the liver to work properly. The use of the term "NAFLD" is used to include all conditions reflecting a form of non-alcohol fatty liver disease, but in particular, NASH.

The term "pharmaceutically acceptable salts" is art-recognized and refers to the relatively non-toxic, inorganic and organic acid addition salts of compounds, including, for example, those contained in compositions of the present invention.

The term "pharmaceutically acceptable carrier" is art-recognized and refers to a pharmaceutically-acceptable material, composition or vehicle, such as a liquid or solid filler, diluent, excipient, solvent or encapsulating material, involved in carrying or transporting any subject composition or component thereof from one organ, or portion of the body, to another organ, or portion of the body. Each carrier must be acceptable in the sense of being compatible with the subject composition and its components and not injurious to the patient.

Some examples of materials which may serve as pharmaceutically acceptable excipients include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) IV fluids, including but not limited to Ringer's solution, 5% dextrose in water, and half normal saline; (19) ethyl alcohol; (20) phosphate buffer solutions; and (21) other non-toxic compatible substances employed in pharmaceutical formulations.

The term "Pharmacological Chaperones " refers to small molecules which stabilize the correct folding of a protein and are administered to the patient resulting in a recovery of function lost due to mutation.

The term "prophylactic" or "therapeutic" treatment is art-recognized and refers to administration to the host of one or more of the subject compositions. If it is administered prior to clinical manifestation of the unwanted condition (e.g., disease or other unwanted state of the host animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

The term "treating" is art-recognized and refers to curing as well as ameliorating at least one symptom of any condition or disease.

NAFLD and NASH in particular, involve the development of histologic changes in the liver that are comparable to those induced by excessive alcohol intake but in the absence of alcohol abuse. Macrovesicular and/or microvesicular steatosis, lobular and portal inflammation, and occasionally Mallory bodies with fibrosis and cirrhosis characterize NAFLD. NAFLD (and NASH in particular) is also commonly associated with hyperlipidemia, obesity, hypertension, atherosclerosis, and Type II diabetes mellitus, commonly known as The Metabolic Syndrome. Other clinical conditions characterized by hepatic steatosis and inflammation include excessive fasting, jejunoileal bypass, total parental nutrition, chronic hepatitis C, Wilson's disease, and adverse drug effects such as those from corticosteroids, calcium channel blockers, high dose synthetic estrogens, methotrexate and amiodarone. Thus, the term "non-alcoholic steatohepatitis" can be used to describe those patients who exhibit these biopsy findings, coupled with the absence of (a) significant alcohol consumption, (b) previous surgery for weight loss, (c) history of drug use associated with steatohepatitis, (d) evidence of genetic liver disease or (e) chronic hepatitis C infection. See, J. R. Ludwig et al., Mayo Clin. Proc, 55, 434 (1980) and E. E. Powell et al., Hepatol., 11, 74 (1990).

Hepatic steatosis (fatty liver) and steatohepatitis (fatty liver with inflammation or scarring) are two forms of the fatty liver disease that develops in children and adults, frequently associated with being overweight or obese. This type of liver disease is often referred to as non-alcoholic steatohepatitis (NASH), because it occurs in the absence of a significant amount of alcohol intake.

One of the features seen in NASH patients is increased plasma and liver lipids. This same feature can be seen in the mice placed on the high fat diet. The high fat diet dramatically increases plasma and liver cholesterol levels compared to chow groups (Figure 2A, C). Mice groups injected with 27-HC do not show altered cholesterol levels.

Plasma and liver triglycerides show varying results. Plasma triglycerides are diminished in Group D, the high fat diet with 27-HC injected mice, when compared to the other high fat diet groups with either control injected for the entire 3 weeks or are only 27-HC injected in the last week (Group C, E; Figure 2B). However, liver triglyceride measurements display no significant differences amongst the high fat diet groups (Groups C-E; Figure 2D). Results for steatosis performed by HE and oil Red O staining also do not show any statistical significance or trends for the high fat diet 27-HC treated groups (Figure 2E, F). Representative pictures from the oil Red O staining showed minimal amounts of fat droplets in the chow groups (Groups A, B) and higher, comparable amounts in the high fat diet groups (Groups C-E).

The fact that hepatic inflammation is reduced by 27-Hydroxycholesterol supplementation was experimentally verified as follows. Immunohistochemical staining was performed for the presence of infiltrated macrophages and neutrophils along with a stain for T-lymphocytes. The staining for infiltrated macrophages, MAC1, and neutrophils, NIMP, show significant differences between the high fat diet group (Group C) in comparison to the remaining high fat diet groups with 27-HC administered for the entire 3 weeks or with control injections for the first 2 weeks and 27-HC injections in the last week (Groups D, E; Figure 3A, 3B). Infiltrated macrophages are significantly decreased in both Group D and E when compared to Group C, the high fat diet with control injections (Figure 3A). Representative pictures display perceptible differences in infiltrated macrophages with large aggregates of positive red stained cells in the high fat diet group and compared to the other four groups. Only Group D mice display decreased neutrophils when compared to Group C (Figure 3B). Pictures from the neutrophil staining also have a prominent display of positive cells in Group C when compared to Groups A, B, D, and E.

To investigate inflammatory changes at the mRNA level due to the differences between the diets, MCP1 gene expression analysis was performed. Real time PCR analysis of MCP1 reveals significant differences when comparing the control injected high fat group (Group C) to the high fat diet with 27-HC injections for three weeks (Group D) and the high fat diet group with 27-HC injections only in the last week (Group E; Figure 3C). This conclusively proves that increased inflammation occurs in the high fat diet group and decreased inflammation in the high fat diet groups with 27-HC supplementation.

We have therewith shown that the exogenous administration of 27-HC can be used to decrease hepatic inflammation.

### Examples

### Example 1: Link between uptake of modified lipids and hepatic inflammation.

To evaluate the involvement of uptake of modified lipoproteins by Kupffer cells (Cs) in the development of diet-induced NASH, female low-density lipoprotein receptor-deficient (Ldlr) knock-out mice were lethally irradiated and transplanted with bone marrow from Msr1/Cd36 double knock-out mice or WT mice and fed a Western diet. Macrophage and neutrophil infiltration revealed that hepatic inflammation was substantially reduced by approximately 30% in Msr1/Cd36 double knock out transplanted mice compared with control mice. Consistent with this, the expression levels of well-known inflammatory mediators were reduced.

Apoptotis and fibrosis were less pronounced in Msr1/Cd36 double knock out transplanted mice, in addition to the protective phenotype of natural antibodies against oxidized low-density lipoprotein in the plasma. Surprisingly, the effect on hepatic inflammation was independent of foam cell formation.

It was concluded that it is not the total amount of lipids in Kupffer cells that will trigger inflammation, but rather the type of lipids or the mechanism of internalization.

### Example 2: Link between hepatic inflammation in NASH and the intracellular fat localization in Kupffer cells.

LdIr knock out mice were depleted from their hematopoietic system by irradiation and were transplanted with bone marrow from mice lacking either Sr-a or Cd36. Control mice were injected with wild-type (Wt) bone marrow. After a recovery period of 9 weeks, the mice were put on a high fat diet (HFD) for 3 months.

It was found that Cd36 knock out and Sr-a⁻ knock out transplanted (tp) mice showed reduced inflammatory markers in blood and liver compared to Wt-tp mice. Moreover, fibrosis was also less pronounced in both Cd36 knock out and Sr-a⁻ knock out tp mice compared to Wt-tp mice. In plasma, the protective IgM auto-antibodies were increased in both Cd36 knock out and Sr-a knock out -tp mice compared to Wt-tp mice. Despite the lack of difference in the appearance of foamy Kupffer cells (KC's), electron microscopy (EM) revealed a significant difference in lysosomal/cytoplasmic cholesterol storage. It was observed that the Wt-tp mice showed increased lysosomal fat whereas the Cd36 knock out - and Sr-a⁻ knock out tp mice showed more cytoplasmic fat inside the KC's.

It was concluded that the reduction in inflammation is correlated with decreased lysosomal cholesterol accumulation in Kupffer cells. These observations establish the link between lysosomal fat accumulation in KC's and hepatic inflammation.

### Example 3: expression of CYP27A1 in Kupffer cells can modulate lysosomal fat accumulation in vitro

We incubated bone marrow derived macrophages from Cyp27A1 knock-out mice and Wt mice with ox LDL for 24 hours. It was found that cholesterol levels in lysosomes were increased. Consequently, cholesterol was significantly more crystallized in macrophages of the Cyp27A1 knock out mice compared to Wt mice.

Since the breakdown of free cholesterol by Cyp27A1 results in the formation of 27-hydroxycholesterol it may be concluded that 27-HC can reduce lysosomal cholesterol accumulation in macrophages.

### Example 4: expression of CYP27A1 in Kupffer cells can modulate lysosomal fat accumulation and thereby hepatic inflammation.

LdIr knock-out mice were transplanted with bone marrow from mice lacking *Cyp27A1.* Control mice were transplanted with wild-type (Wt) bone marrow. After a recovery period of 9 weeks, the mice were put on chow or high fat high cholesterol (HFC) diet for 3 months.

It was found that Kupffer cells of the *Cyp27A1*^{*-*/}*⁻-*tp mice showed increased lysosomal fat accumulation. Additionally, cholesterol was more crystallized in *Cyp27A1*^{*-*/}*⁻-*tp mice compared to *Wt*-tp mice. Surprisingly, CD68 staining revealed that KC's were less foamy upon HFC feeding in *Cyp27A1*^{*-*/}*⁻-*tp mice. Liver histology demonstrated that *Cyp27A1*^{*-*/}*⁻-*tp mice had increased hepatic inflammation compared to *Wt-*tp mice as indicated by the elevated numbers of infiltrated macrophages, neutrophils and T-cells. These findings were confirmed by hepatic expression of *Tnf, II-1b* and *II-6.*

It was concluded that CYP27A1 plays an important role in liver inflammation in NASH, in particular in diet-induced NASH.

It may therefore be concluded that there is a clear association between lysosomal fat accumulation and hepatic inflammation and that inflammation may be decreased by increasing the level or activity of 27-HC.

### Example 5: Liver inflammation may be ameliorated or treated by administration of 27-HC to a mammal

To determine whether exogenous 27-HC can reduce hepatic inflammation, hyperlipidemic LDLR knock-out mice may be fed either chow, HFC diet or HFC diet supplemented with 27-hydroxycholesterol (0.1%w/w) for 3 weeks. Then, the mice may be sacrificed and detailed characterization of liver and plasma may be performed.Mice receiving diet supplemented with 27-HC will show less lysosomal cholesterol accumulation and reduced liver and systemic inflammation, compared to mice receiving HFC diet.

### Example 6: Materials and methods used in the examples Mice

Female *LdIr*^{*-*/*-*} mice were lethally irradiated and transplanted with *Wt, Cd36*^{*-*/*-*} and *Msr*^{*-*/*-*} bone marrow. After a recovery period of 9 weeks, the mice were given a HFC diet for 3 months (n=9 in Wt-tp mice, n=8 in both Cd36-/--tp and Msr1-/--tp mice).

Collection of blood, specimens, biochemical determination of lipids in plasma and liver, liver histology, RNA isolation, cDNA synthesis and qPCR, determination of chimerism, aminotransferases, oxysterols and auto-antibody titers against modified LDL are described previously (Bieghs, V., et al.,. Gastroenterology, 2010).

### Blood leukocyte analysis

Sixty microliter of total blood was washed extensively in erythrocyte lysis buffer (0.155 M NH₄CL, 10 mM NaHCO₃) for leukocyte analysis. Cells were stained for monocytes (Mac1-PE), granulocytes (Gr1-FITC), B-cells (6B2-PE) and T-cells (KT3-FITC) (BD Sciences Pharmingen, San Diego, California, USA) in PBS containing 5% normal mouse serum and 1% FCS. After 1 hour of incubation, cells were washed and analyzed by FACS analysis (Facssort, BD Sciences Pharmingen).

### Electron microscopy

Detailed overview about (post)fixation, embedding, cutting and type of electron microscope is described previously (Hepatology). Scoring of the electron microscopy pictures is performed by a trained specialist. Fifty Kupffer cells per mouse were analyzed and scored. The Kupffer cells were divided into five different categories, e.g. primary lysosomes, secondary lysosomes, fatty lysosomes, cytoplasmic fat accumulation and degenerated lysosomes and the percentage of each subtype is calculated. Furthermore, the secondary lysosomes were scored from 0 to 3 for their fat content and complexicity, where score 0 indicates not positive and score 3 indicates extreme fat accumulation inside the lysosomes of the KCs / extremely complex indicated by electrodense particles. The same scoring index is used for the cytoplasmic fat accumulation inside Kupffer cells.

### Statistical analysis

Data was statistically analyzed by performing one-way ANOVA test with a Bonferroni post-test using GraphPad Prism for comparing *Wt*-tp, *Cd36*^{*-*/-}-tp and *Msr*^{*-*/-}-tp mice with each other. Data were expressed as the mean ± SEM and considered significant at p<0.05. *, ** and *** indicate p < 0.05, 0.01 and 0.001 resp.

### Example 7: NASH treatment with 27-HC

Our experimental approach consists of utilizing low-density (LDL) receptor deficient mice (LDLr-/-) on a C57BI6 background. This mouse model is predisposed to elevated serum cholesterol levels when fed a high fat diet. On a normal chow diet these mice have a threefold increase in cholesterol levels when compared to controls (39). Our lab has determined that two weeks is sufficient for mice to reach a steatotic and inflamed liver state.

Experiments were performed in accordance with Dutch law for animal experimentation and approved by the Committee for Animal Welfare of the University of Maastricht. In this study we aim to investigate the ability of 27-hydroxycholesterol (generous gift from Dr. Norman B. Javitt; New York University School of Medicine) to reduce hepatic inflammation and to characterize its potential use as a therapeutic agent for NASH patients. This will be achieved through the use of low-density lipoprotein (LDL) receptor deficient (LDLr-/-) female mice on a C57BI6 background, ranging from the ages of 10 to 12 weeks.

A total of five different experimental groups, ranging from 8 to 9 mice per group, were used to assess the effects of 27-HC. The 27-HC was dissolved in 2-hydroxypropyl-β-cyclodextrin (Sigma-Aldrich GmbH H5784; St. Louis, MO) and injected with a concentration of 40 mg of 27-HC per kilogram of body weight via a subcutaneous injection. Mice in Group A received a chow diet and control injections consisting of 2-hydroxypropyl-β-cyclodextrin dissolved in sodium chloride. Group B mice were also on the chow diet but are given daily subcutaneous injections of 27-HC. Group C were the mice that received the high fat diet (21% fat; 0.2% cholesterol) and control injections. To differentiate between a therapeutic and a regression effect of the 27-HC, the following two groups were setup: a high fat diet with daily 27-HC injections (Group D) and a high fat diet group with two weeks of control injections followed by a week of 27-HC injections (Group E). Mice were housed under standard condition with food and water available ad libidum.

One day previous to the start of the experiment the mice were weighed and 100-200 µl of blood were collected in a capillary tube after four hours of fasting via an incision in the tail vein. On day 0 the feeding of specialized diets and injections began according to each group. On the 21st day after the start of the experiment, the animals were weighed and fasted for four hours and had another 100-200µl of blood drawn and collected in a capillary tube via an incision in the tail vein. Afterwards the mice were sacrificed via cervical dislocation and immediately had their livers removed via a laparotomy consisting of an incision from the pelvis to the rib cage to gain access into the abdomen. The livers were then dissected into several pieces and were either preserved in liquid nitrogen for storage at -80°C, or in 4% formaldehyde for tissue fixation.

### Example 8: Liver Lipid Analysis

Homogenization of approximately 50 mg of frozen liver tissue was completed at 5000 rpm for 30 seconds with 1.0 mm glass beads along with 1.0 ml SET Buffer (sucrose 250 mmol/L, EDTA 2 mmol/L, and Tris 10 mmol/L). A total of three freeze-thaw cycles and passage three times through a 27-gauge syringe needle ensured cell destruction. The homogenate was used to measure protein, triglyceride, and cholesterol content. Protein material was diluted 20 times. To measure the protein content, 25 µl of the diluted sample was pipetted into a flat bottom micro-plate. After adding 200 µl of the reagents (50 parts A, 1 part B) and letting the mixture stand for 30 minutes at 37°C, the absorbance was measured utilizing the enzymatic color test bicinchoninic acid (BCA) method (Pierce, Rockford, IL) at a wavelength of 560 nm with a Benchmark 550 Micro-plate reader (170-6750XTU; Bio-Rad, Hercules, CA).

For triglycerides (diluted 5 times) and cholesterol (undiluted) measurements, 7.5 µl of homogenate were pipetted onto their respective flat bottom measure plates in which 200 µl of their respective reagents were mixed in and set to incubate for 45 minutes at room temperature (Roche GPO-PAP kit). Both triglycerides and cholesterol had their absorbencies read at 490 nm. All of the protocols were followed according to manufacturer's instructions and read on a Benchmark 550 Micro-plate reader (170-6750XTU; Bio-Rad, Hercules, CA). Concentrations of triglycerides and cholesterol were set relative to the protein content of the sample; i.e. lipid component divided by protein, divided by 100 giving the actualized concentration in mg/dl.

### Example 9: Plasma Lipid Analysis

Plasma cholesterol and triglyceride measurements were tested with the enzymatic color tests. Diluted cholesterol serum plasma samples (2 times dilution for control diet; 15 times dilution for high fat diet) were pipetted in 7.5 µl volumes into a flat bottom micro-plate. Then, 200 µl of the cholesterol reagent was added, mixed, and incubated at room temperature for 45 minutes. Absorbance was measured at the 490 nm wavelength with a Benchmark 550 Micro-plate reader. For the triglyceride portion, the high fat diet samples were diluted twice whereas the control diet did not need to be diluted. Again, 7.5 µl of each sample was pipetted into a flat bottom micro-plate. Subsequently, 200 µl of reagent A was added, mixed, and after 15 minutes of incubation read at a wavelength of 545 nm for the initial absorbance. Another 40 µl of reagent B was added for 30 minutes of incubation at room temperature. The final absorbance was then measured at 545 nm. The concentration of triglycerides was then calculated by subtracting the initial concentrations from the final concentrations. All protocols were followed according to the manufacturer's regulations (cholesterol CHOD-PAP; 1489232; Roche, Basel, Switzerland; serum triglyceride determination kit, TR0100; Sigma-Aldrich; NEFAC, ACS-ACOD, 999-75406; Wako Chemicals, Neuss, Germany) and a Benchmark 550 Micro-plate Reader (170-6750XTU; Bio-Rad, Hercules, CA) was used to determine the measurements.

### Example 10: Immunohistochemistry and Histological Analysis

Liver tissues were fixated in formaldehyde, embedded in paraffin, and cut into 4 µm sections. A hematoxylin and eosin (HE; Hematoxilin 4085.9002; Klinipath Duiven, The Netherlands; and Eosin, E4382; Sigma-Aldrich) stain was performed to assess the general condition of the liver including hepatic inflammation, fat accumulation, and the foamy appearance of Kupffer Cells. First, the slides had the paraffin removed with xylol and descending concentrations of ethanol from 100% to 50%. Next, the samples were colored for 10 seconds in hematoxylin, washed, and then dipped in eosin staining solution for 3 minutes. Dehydration of the slides was obtained through several washings in ascending degrees of ethanol, 70% to 100% (Sigma-Aldrich Co. St. Luis, MO). Slides were preserved by adding two drops of Entellan (Merk KGaA; Darmstadt, Germany) and a glass cover slip. The hematoxylin dye gave the nuclei a purple/blue hue and eosin provided the cytoplasm a pink/red hue enabling visual differentiation.

Stained sections were scored in a blind manner by a specialized animal pathologist. In regards to inflammation, a score of zero denoted no aggregates of inflammatory cells in the tissue; a score of one was designated for moderate cell infiltration, a score of two for large amounts of cells aggregates, and a score of three for large clusters of inflammatory marked cells over the entire tissue. Sections were scored for the quantity of fat droplets and for the foamy appearance of Kupffer cells. A scoring system of 0 to 3 was used to designate minimal amounts to severe steatosis and the abundant foamy appearance of Kupffer cells.

To distinguish between inflammatory cell types and further demonstrate their presence, immunohistochemical staining for T-lymphocytes (CD3), Kupffer cells (CD68), infiltrated macrophages (MAC1), and neutrophils (NIMP) were completed utilizing 7 µm thick frozen liver sections. Hydrogen peroxide was utilized to block endogenous peroxidase activity, and 4% fetal calf serum was used to block nonspecific binding sites. CD3 positive cells were stained with a polyclonal rabbit anti-human CD3 antibody (dilution 1:200), CD68 positive cells with monoclonal mouse anti-human antibody (dilution 1:100; CD3 and CD68 antibodies were generous gifts from Prof Kraal, Free University, Amsterdam, The Netherlands), and MAC1 positive cells with polyclonal rabbit anti-human MAC1 antibody (DAKO R0841; dilution 1:1000). For the CD3, CD68, and MAC1 stains the ABC-kit was used for amplification (first incubations: Avidin D 1:5 dilution, Biotin 1:5 dilution; second incubations: Avidin D 1:50 dilution, Biotin 1:50 dilution; Vector Laboratories Inc. Burlingame, CA) The NIMP stain for neutrophils does not require amplification and uses a rat-anti-mouse Ly6-C, clone NIMPR14 (dilution 1:100; again a gift from Prof Kraal). The secondary antibody applied to the CD3, CD68, and MAC1 stains was a biotinylated polyclonal rat anti-rabbit IgG (DAKO E0468; 1:300 diltuion). The NIMP stain has a polyclonal rabbit anti-rat immunoglobulin horse radish peroxidase (DAKO E0450 1:100 dilution) secondary antibody. The detection kit AEC (3-Amino-9-ethylcarbazole; A85SK-4200.S1; Bio-connect, Huissen, The Netherlands) was used as a color substrate for all of the stains to identify the positive cells, and hematoxylin (4085.9002, Klinipath, Duiven, The Netherlands) was applied as nuclear counter stain. All sections were preserved with two drops aqueous mounting medium (S302580; DAKO, Glostrup, Denmark) and a glass cover slip.

To verify the quantification of fat, 7 µm thick frozen sections were stained with oil red O (ORO; 00725; Sigma-Aldrich) the neutral lipid marker. First, the samples were fixated for one hour in 3.7% formaldehyde. These were then stained with 0.2% oil red utilizing triethyl-phospate (60%) as the solvent for 30 minutes. Mayer's hematoxylin (4085.9002, Klinipath, Duiven, The Netherlands) was used as the counterstain for the sections, rinsed under running water, and preserved with a glass cover slip using 10% glycerol in PBS. The lipid droplets stained red were quantified on a scale of 0 to 3. A score of 0 was designated to those samples with very few indications of lipid deposits, a score of one for mild amounts, a score of two for moderate amounts, and a score of 3 for severe steatosis.

Nikon digital camera model DMX1200 and ACT-1 v2.63 software from Nikon Corporation were used to photograph each section. For the immunohistochemical staining six pictures were randomly taken of each sample and for the oil red O stain four pictures were randomly taken both at a 200x magnification. The positive cells for each picture were then counted or scored and noted as cells or lipid droplets per square millimeter respectively.

### Example 11: RNA extraction, cDNA synthesis and RT-PCR

Approximately 25 mg of frozen liver tissue was used as the source for total RNA extraction. Homogenization of the frozen liver tissue was completed at 5000 rpm for 30 seconds with 1.0 mm glass beads along with 1.0 ml Tri Reagent (Sigma Aldrich T9424) to disassociate nucleoprotein complexes. After allowing samples to stand for 5 minutes, 200 µl of chloroform were pipetted into each of the tubes. These were then shaken vigorously, left to rest for 10 minutes, and then centrifuged at 12000x g for 15 minutes at 4°C. The RNA from the upper aqueous phase was delicately removed and placed into a new tube. To this new tube, 0.5 ml of isopropanol was added and then the mixed sample was centrifuged for 10 minutes again at 4°C. After removing the supernatant, the RNA pellet that precipitated was resuspended in 0.5 ml of 70% ethanol and again centrifuged for 5 minutes at 4°C. The pellet was again isolated and redissolved in 200 µl sterile water.

In order to verify the purity and concentration of the isolated RNA, the NanoDrop program (NanoDrop 1000 V3.1.2; NanoDrop Technologies Montchanin, DE) was used. A drop, 1.5 µl, of each sample was measured by the NanoDrop device. After confirmation of purity and rendering pipettable concentrations, the master mix of iScript mix (4 µl per sample) and iScript Reverse Transcriptase (1 µl per sample) were added to the mix plate along with 15 µl (500 ng per reaction) of isolated RNA from each sample (Bio-Rad Hercules, CA). This plate was then placed in the thermal PCR cycler to generate cDNA.

Samples were plated onto a 384 PCR well plate for the testing of the following genes: CD68, MCP1, IL-1β, TNFα, ICAM, and Cyclophilin A. Along with the 5 µl of each cDNA sample (2 ng), Master Mix (10 µl IQ SYBR Green Supermix with fluoresceine), primers (0.8 µl) and sterile water (4.2 µl) were added for each sample specific for each gene. For the targeted genes, the primers were developed by using Primer Express v2.0 (Applied Biosystems, Foster City, CA) utilizing the default settings. Cyclophylin A (Ppia) was the endogenous control gene used to standardize the amount of cDNA made (Table 1). SDS ABi 7900HT with PowerSybr Green mastermix (4329001and 4368708; both Applied Biosystems, Foster City, CA) were used to perform the real-time PCR. This was completed according to manufacturer's protocols by utilizing chemicals from Sigma Chemical Co. The PCR results were analyzed using the relative standard curve method.

**Table 1: Primer sequences used in RT-PCR.**

| **Gene** | **Primer Forward** | **Primer Reverse** |
|---|---|---|
| CD68 | TGACCTGCTCTCTCTAAGGCTACA (SEQ ID NO: 1) | TCACGGTTGCAAGAGAAACATG (SEQ ID NO: 7) |
| ICAM | TTCTTGGTGGTGAGCCTGAGAAGAG (SEQ ID NO: 2) | CAAGTACCTGGCTGTGCAGATTAG (SEQ ID NO: 8) |
| IL-1β | AGAATGAGCTGTTATTTGAGGTTGATG (SEQ ID NO: 3) | GTGAGAAATCTGCAGCTGGATGT (SEQ ID NO: 9) |
| MCP-1 | CATAGCAGCCACCTTCATTCC (SEQ ID NO: 4) | TCTGCACTGAGATCTTCCTAT(SEQ ID NO: 10) |
| TNFα | CATCTTCTCAAAATTCGAGTGACAA (SEQ ID NO: 5) | TGGGAGTAGACAAGGTACAACCC (SEQ ID NO: 11) |
| Ppia | TTCCTCCTTTCACAGAATTATTCCA (SEQ ID NO: 6) | CCGCCAGTGCCATTATGG (SEQ ID NO: 12) |

### Example 12: Data Analysis

Results are presented as mean standard error of the mean (± SEM). Differences between groups were assessed by ANOVA and significant effects were analyzed by post hoc Bonferroni corrections. These differences are considered statistically significant at P<0.05 indicated by one asterisk, a P-value of <0.01 with two asterisks, and three asterisks indicating a significance level of P<0.001. All analyses were performed using a commercially available statistics package (GraphPad Prism version 5; GraphPad Software Inc, San Diego, CA, U.S; www.graphpad.com).

### SEQUENCE LISTING

<110> Universiteit Maastricht
<120> METHOD FOR TREATING FATTY LIVER DISEASES, IN PARTICULAR NON-ALCOHOLIC STEATOHEPATITIS
<130> 171 WO
<150> 10172304.7
   <151> 2010-08-09
<160> 12
<170> PatentIn version 3.5
<210> 1
   <211> 24
   <212> DNA
   <213> mus musculus
<400> 1
   tgacctgctc tctctaaggc taca
   24
<210> 2
   <211> 25
   <212> DNA
   <213> mus musculus
<400> 2
   ttcttggtgg tgagcctgag aagag
   25
<210> 3
   <211> 27
   <212> DNA
   <213> mus musculus
<400> 3
   agaatgagct gttatttgag gttgatg
   27
<210> 4
   <211> 21
   <212> DNA
   <213> mus musculus
<400> 4
   catagcagcc accttcattc c
   21
<210> 5
   <211> 25
   <212> DNA
   <213> mus musculus
<400> 5
   catcttctca aaattcgagt gacaa
   25
<210> 6
   <211> 25
   <212> DNA
   <213> mus musculus
<400> 6
   ttcctccttt cacagaatta ttcca
   25
<210> 7
   <211> 22
   <212> DNA
   <213> mus musculus
<400> 7
   tcacggttgc aagagaaaca tg
   22
<210> 8
   <211> 24
   <212> DNA
   <213> mus musculus
<400> 8
   caagtacctg gctgtgcaga ttag
   24
<210> 9
   <211> 23
   <212> DNA
   <213> mus musculus
<400> 9
   gtgagaaatc tgcagctgga tgt
   23
<210> 10
   <211> 21
   <212> DNA
   <213> mus musculus
<400> 10
   tctgcactga gatcttccta t
   21
<210> 11
   <211> 23
   <212> DNA
   <213> mus musculus
<400> 11
   tgggagtaga caaggtacaa ccc
   23
<210> 12
   <211> 18
   <212> DNA
   <213> mus musculus
<400> 12

### References

1. Ludwig J, Viggiano TR, McGill DB, Oh BJ. Nonalcoholic steatohepatitis: Mayo Clinic experiences with a hitherto unnamed disease. Mayo Clin Proc. 1980 Jul;55(7):434-8.
2. National Digestive Diseases Information Clearinghouse N. Nonalcoholic Steatohepatitis. Bethesda, MD: NIH; 2011 [updated 2011; cited]; A Service of the National Institue of Diabetes and Digestive and Kidney Diseases (NIDDK), NIH]. Available from: http://www.digestive.niddk.nih.gov/ddiseases/pubs/nash/.
3. Marra F, Gastaldelli A, Svegliati Baroni G, Tell G, Tiribelli C. Molecular basis and mechanisms of progression of non-alcoholic steatohepatitis. Trends Mol Med. 2008 Feb; 14(2): 72-81.
4. Sheth SG, Gordon FD, Chopra S. Nonalcoholic steatohepatitis. Ann Intern Med. 1997 Jan 15;126(2):137-45.
5. Vernon G, Baranova A, Younossi ZM. Systematic review: the epidemiology and natural history of non-alcoholic fatty liver disease and non-alcoholic steatohepatitis in adults. Aliment Pharmacol Ther. May 30.
6. Low S, Chin MC, Deurenberg-Yap M. Review on epidemic of obesity. Ann Acad Med Singapore. 2009 Jan;38(1):57-9.
7. Mendler M.D. M. Fatty Liver: Nonalcoholic Fatty Liver Disease (NAFLD) and Nonalcoholic Steatohepatitis (NASH). 2010 [updated 2010; cited]; 4]. Available from: http://www.medicinenet.com/fatty_liver/page4.htm#toci.
8. Malik SM, Devera ME, Fontes P, Shaikh O, Sasatomi E, Ahmad J. Recurrent disease following liver transplantation for nonalcoholic steatohepatitis cirrhosis. Liver Transpl. 2009 Dec;15(12):1843-51.
9. Ong JP, Younossi ZM. Epidemiology and natural history of NAFLD and NASH. Clin Liver Dis. 2007 Feb;11(1):1-16, vii.
10. Pascale A, Pais R, Ratziu V. An overview of nonalcoholic steatohepatitis: past, present and future directions. J Gastrointestin Liver Dis. Dec;19(4):415-23.
11. Kaplan MM. Patient information: Nonalcoholic steatohepatitis (NASH). 2010 [updated 2010; cited]; Available from: http://www.uptodate.com/contents/patient-information-nonalcoholic-steatohepatitis-nash.

## Claims

1. Composition comprising an effective amount of 27 hydroxycholesterol or a pharmaceutically acceptable salt thereof for use in the prevention or treatment of liver inflammation in non-alcoholic fatty liver disease.

2. Composition for use according to claim 1 wherein the non-alcoholic fatty liver disease is non-alcoholic steatohepatitis.

3. Composition for use according to claims 1 or 2 wherein the treatment is part of a combination treatment for metabolic syndrome.

4. Composition for use according to claims 1 - 3 wherein the composition comprises a pharmaceutically acceptable carrier.

5. Composition for use according to claims 1 - 4 wherein the composition is a food composition or a food supplement.

## Patentansprüche

1. Zusammensetzung, die eine wirksame Menge an 27-Hydroxycholesterol oder eines pharmazeutisch unbedenklichen Salzes davon umfasst, zur Verwendung in der Vorbeugung oder Behandlung von Leberentzündung bei nichtalkoholischer Fettleberkrankheit.

2. Zusammensetzung zur Verwendung nach Anspruch 1, wobei es sich bei der nichtalkoholischen Fettleberkrankheit um nichtalkoholische Steatohepatitis handelt.

3. Zusammensetzung zur Verwendung nach Anspruch 1 oder 2, wobei die Behandlung Teil einer Kombinationsbehandlung für metabolisches Syndrom ist.

4. Zusammensetzung zur Verwendung nach den Ansprüchen 1-3, wobei die Zusammensetzung einen pharmazeutisch unbedenklichen Träger umfasst.

5. Zusammensetzung zur Verwendung nach den Ansprüchen 1-4, wobei es sich bei der Zusammensetzung um eine Nahrungsmittelzusammensetzung oder ein Nahrungsergänzungsmittel handelt.

## Revendications

1. Composition comprenant une quantité efficace de 27 hydroxycholestérol ou d'un sel pharmaceutique non acceptable de celui-ci pour l'utilisation dans la prévention ou le traitement de l'inflammation du foie dans le cas d'une stéatose hépatique non alcoolique.

2. Composition pour l'utilisation selon la revendication 1, dans laquelle la stéatose hépatique non alcoolique est la stéatohépatite non alcoolique.

3. Composition pour l'utilisation selon les revendications 1 ou 2, dans laquelle le traitement fait partie d'un traitement combiné pour le syndrome métabolique.

4. Composition pour l'utilisation selon les revendications 1 à 3, dans laquelle la composition comprend un support pharmaceutiquement acceptable.

5. Composition pour l'utilisation selon les revendications 1 à 4, dans laquelle la composition est une composition alimentaire ou un supplément alimentaire.
